# EUROPEAN PATENT APPLICATION

(11) **EP 3 616 660 A1**
(43) Date of publication of application: **04.03.2020**
(21) Application number: 18851455.8
(22) Date of filing: 22.06.2018
(51) Int. Cl.: A61F 5/455, A61F 5/44

(54) **EASILY INSERTED MENSTRUAL CUP**

(30) Priority: 30.08.2017 KR 20170110338
(71) Applicant: Loon Lab Inc., Seongnam-si, Gyeonggi-do 13466 (KR)
(72) Inventor: HWANG, Ryong, Cheonan-si Chungcheongnam-do 31207 (KR)
(74) Representative: RGTH
(86) International application number: PCT/KR2018/007115
(87) International publication number: WO 2019/045239

(57) **Abstract**

The present invention relates to an easily inserted menstrual cup. The menstrual cup according to an embodiment of the present invention includes: a main body having an opening formed at one side and a storage part for accommodating menstrual blood therein formed at an inner side; a rim formed at the opening of the main body and configured to prevent leakage of the menstrual blood by closely contacting with the wall of the vagina when the menstrual cup is inserted into the vagina; and a frame formed inside a wall of the main body, wherein the frame is formed of a shape memory material.

## Description

### FIELD OF THE INVENTION

The present invention relates to an easily inserted menstrual cup, and more particularly, to a menstrual cup capable of being deformed to an easy-to-insert shape.

### BACKGROUND

Menstrual blood leakage prevention tools such as pads, menstrual cups, and tampons are used to prevent menstrual blood from leaking out of the vagina to help women feel more comfortable during menstruation.

Among the menstrual blood leakage prevention tools, menstrual cups are generally larger in capacity than pads and tampons and have almost no leakage of menstrual blood. Also, unlike pads and tampons, menstrual cups are reusable so that they are economical and eco-friendly. Also, since menstrual cups are used by being inserted into the body, one can freely participate in exercise or activity such as swimming even when wearing a menstrual cup. Furthermore, menstrual cups have been used for a long time and been proven to be safe.

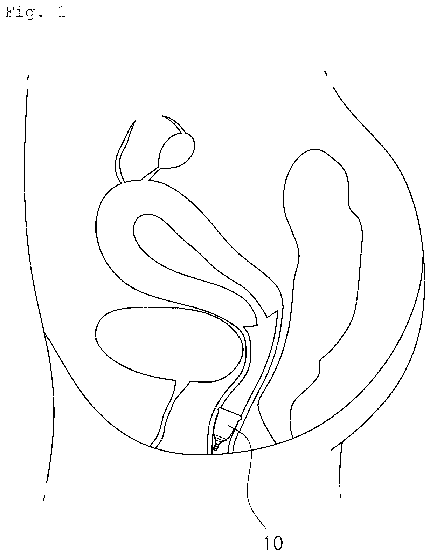

FIG. 1 is a view illustrating a state in which a menstrual cup is inserted into the body. A menstrual cup 10 is inserted into the vagina and fixed in a direction in which an opening of the menstrual cup 10 faces the inside of the body so that the menstrual cup 10 can hold menstrual blood therein. Specifically, when the opening of the menstrual cup 10 is folded and inserted into the vagina, the folded opening is unfolded due to elasticity, and the unfolded opening comes into contact with the wall of the vagina. Here, a negative pressure is formed inside the menstrual cup 10 such that the menstrual cup 10 is fixed.

In this way, in order to insert a menstrual cup into the vagina, the menstrual cup should be folded and inserted. Various methods such as a C-fold, V-fold, 7-fold, and push-down are known as methods of folding a menstrual cup. However, in order to give elasticity to a menstrual cup, the menstrual cup should be manufactured using an elastic material (e.g., silicone or rubber) having a predetermined thickness, and thus, even when the menstrual cup is folded according to the above methods, there is a limitation in reducing the size of the menstrual cup, and many users still feel a feeling of repulsion or experience discomfort about inserting a menstrual cup. Further, in a case in which a menstrual cup is manufactured to be thin in order to reduce such discomfort, due to insufficient elasticity, the menstrual cup may not be properly placed in the vagina after being inserted thereinto, and leakage of menstrual blood may occur in some cases.

In the case of tampons which are used by being inserted into the vagina like menstrual cups, the tampons may be easily inserted without a feeling of repulsion or discomfort by using an applicator. However, since menstrual cups are formed of a material having elasticity, thus making it difficult for the menstrual cups to maintain a folded state, it is substantially impossible to apply the applicator to the menstrual cups.

### SUMMARY OF THE INVENTION

An objective of the present invention is to solve the above-described problems. Further, the present invention is directed to providing a menstrual cup capable of being deformed to an easy-to-insert shape, thereby being easily inserted without a feeling of repulsion or discomfort about inserting the menstrual cup.

An easy-to-insert menstrual cup according to one embodiment of the present invention includes: a main body having an opening formed at one side and a storage part for accommodating menstrual blood therein formed at an inner side; a rim formed at the opening of the main body and configured to prevent leakage of the menstrual blood by closely contacting with the wall of the vagina when the menstrual cup is inserted into the vagina; and a frame formed inside a wall of the main body, wherein the frame is formed of a shape memory material.

The menstrual cup according to one embodiment of the present invention may further include a ring part formed inside the rim in a circumferential direction of the rim, wherein the ring part may be formed of a shape memory material.

According to one embodiment of the present invention, the frame formed inside the wall of the main body and the ring part formed inside the rim may be integrally formed.

According to one embodiment of the present invention, the rim may be formed of an auxetic structure having a negative Poisson's ratio.

According to one embodiment of the present invention, the frame may be formed in a longitudinal direction of a wall surface of the main body extending from the opening of the main body to the opposite side of the main body.

According to one embodiment of the present invention, the frame may be formed of nitinol.

According to one embodiment of the present invention, the frame may be restored to its original shape at body temperature, or the frame may be deformed at room temperature and then restored to its original shape at a temperature higher than body temperature.

According to one embodiment of the present invention, an inner space may be provided at the opposite side of the opening of the main body, and a power supply device may be formed in the inner space to supply electrical energy to the frame.

According to one embodiment of the present invention, a protrusion may be formed on an inner wall of the main body, and the frame may be disposed inside the protrusion. Here, the protrusion may be formed such that a thickness of the protrusion on an outer side of the frame is larger than a thickness of the protrusion on an inner side of the frame.

According to one embodiment of the present invention, a menstrual cup can be deformed to an easy-to-insert shape, the volume of the menstrual cup can be significantly reduced after the deformation, and the deformed state of the menstrual cup can be maintained in a process of inserting the menstrual cup. Therefore, a user can easily insert the menstrual cup into the vagina without a feeling of repulsion and discomfort about inserting the menstrual cup.

### BRIEF DESCRIPTION OF THE DRAWINGS

FIG. 1 is a view illustrating a state in which a menstrual cup is inserted into the body.
FIG. 2 is a perspective view of a menstrual cup according to a first embodiment of the present invention.
FIG. 3 is a partially see-through view of the menstrual cup according to the first embodiment of the present invention.
FIG. 4 is a view illustrating an example of a folded shape of the menstrual cup according to the first embodiment of the present invention which is folded to be inserted into the vagina.
FIG. 5 is a partially see-through view of a menstrual cup according to a second embodiment of the present invention.
FIG. 6 is a partially see-through view of a menstrual cup according to a third embodiment of the present invention.
FIG. 7 is a perspective view of a menstrual cup according to a fourth embodiment of the present invention.

### <Description of reference numerals>

100, 200, 300, 400: menstrual cup
110, 210, 310, 410: main body
111, 211, 311: frame
115: protrusion
120, 220, 320, 420: rim
221, 321: ring part
130, 230, 330, 430: handle

### DETAILED DESCRIPTION

Hereinafter, exemplary embodiments of the present invention will be described in detail with reference to the accompanying drawings to an extent that those of ordinary skill in the art to which the present invention pertains can easily practice the present invention.

To clearly describe the present invention, description of parts not related to the present invention will be omitted, and the same elements will be denoted by the same or similar reference numerals throughout. Also, since the size, thickness, position, and the like of each element illustrated in the drawings are arbitrarily shown for convenience of description, the present invention is not necessarily limited to those illustrated in the drawings. That is, it should be understood that specific shapes, structures, and features described herein may be implemented by being modified from one embodiment to another embodiment without departing from the spirit and scope of the present invention, and the position or arrangement of each element may also be modified without departing from the spirit and scope of the present invention.

Therefore, the detailed description given below should not be construed as limiting, and the scope of the present invention should be construed as encompassing the scope of the claims below and their equivalents.

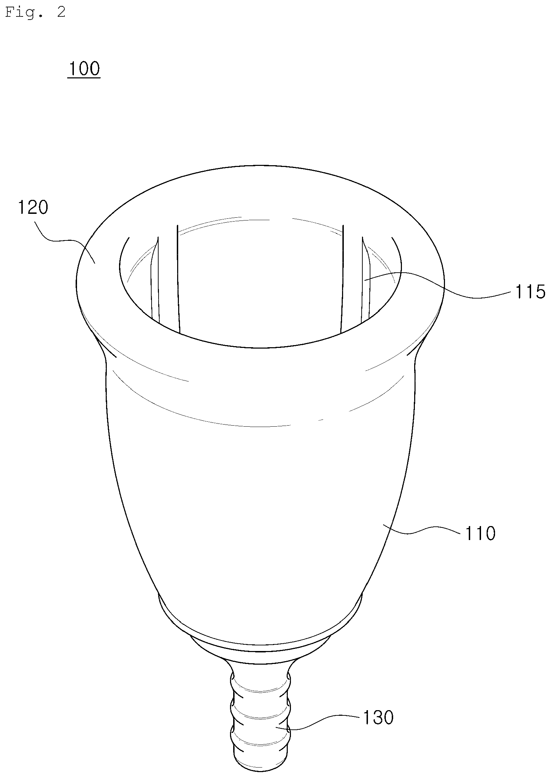

FIG. 2 is a perspective view of a menstrual cup according to a first embodiment of the present invention. Referring to FIG. 2, a menstrual cup 100 according to the first embodiment of the present invention may include a main body 110, a rim 120, and a handle 130.

The main body 110 of the menstrual cup 100 is formed in the shape of a cup and has a storage part, which is capable of accommodating menstrual blood, formed therein. In the present embodiment, a form in which a diameter of the main body 110 gradually increases from a portion where the main body 110 is connected to the handle 130 to a portion where the main body 110 is connected to the rim 120 is described as an example. However, the present invention is not limited thereto, and the shape of the main body may be changed to various other shapes capable of holding menstrual blood therein. Also, the main body may be formed in various sizes according to the capacity of menstrual blood to be stored.

The rim 120 of the menstrual cup 100 is formed at an opening of the main body 110 and may serve to prevent leakage of menstrual blood by pressing against the wall of the vagina when the menstrual cup 100 is inserted into the vagina. In the present embodiment, the rim 120 is illustrated as being formed in a circular shape. However, unlike this, the rim 120 may also be formed in other shapes such as an elliptical shape and a polygonal shape.

The handle 130 of the menstrual cup 100 may be formed to protrude from an opposite side to the opening of the main body 110 and may serve to facilitate use of the menstrual cup 100 when a user inserts the menstrual cup 100 into the vagina or removes the menstrual cup 100 from the vagina. When a length of the handle 130 is too long, a user may feel uncomfortable when participating in an activity after inserting the menstrual cup 100. On the contrary, when the menstrual cup 100 is too short, a user may have difficulty in removing the menstrual cup 100. Thus, the handle 130 may be formed to have a proper length in consideration of such circumstances. Since a length of the handle that makes a user feel uncomfortable and a length of the handle that is required for easily removing a menstrual cup may be different for each user, the handle may be formed to be long and may be cuttable so that the user may directly adjust a length of the handle.

Meanwhile, in the present embodiment, a case in which the handle 130 of the menstrual cup 100 is formed in the shape of a column is described as an example. However, the shape of the handle 130 may be changed to other shapes such as a ring shape and a spherical shape, or a menstrual cup may also be formed without a handle.

The main body 110, the rim 120, and the handle 130 of the menstrual cup 100 according to the present embodiment may be integrally formed and formed of a material such as silicone or rubber which has excellent elasticity and is non-toxic.

According to the first embodiment of the present invention, a protrusion 115 may be formed on an inner wall of the main body 110 of the menstrual cup 100. The protrusion 115 is a structure for inserting a frame, which will be described below, therein. The protrusion 115 may be formed to extend in a direction from the rim 120 toward the handle 130. In the present embodiment, the protrusion 115 is provided as four protrusions 115 formed at equal intervals in a circumferential direction on the inner wall. However, the number of protrusions on the inner wall of the main body and intervals at which the protrusions are disposed are not limited thereto in the present invention.

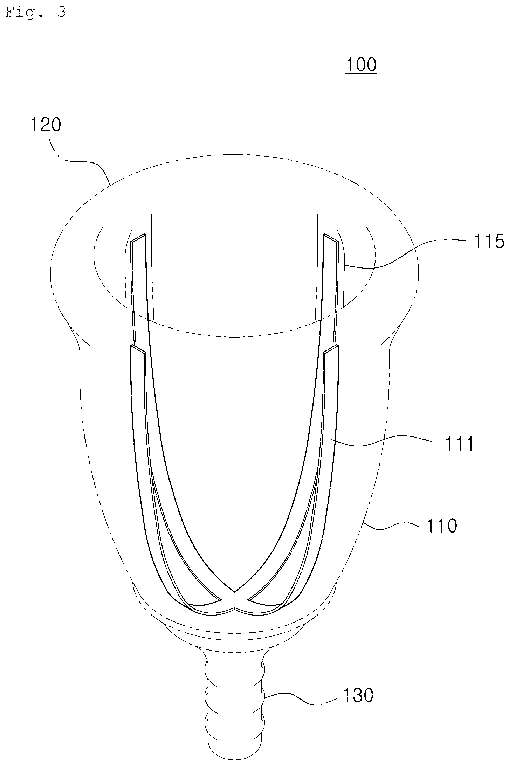

FIG. 3 is a partially see-through view of the menstrual cup according to the first embodiment of the present invention. Referring to FIG. 3, in the menstrual cup 100 according to the present embodiment, a frame 111 is formed in the protrusion 115 formed on the inner wall of the main body 110. The frame 111 may be formed as a thin metal strip and disposed in the protrusion 115 so as to extend in a longitudinal direction of the main body 110, i.e., the direction from the rim 120 toward the handle 130. The frame 111 may be provided as four frames 111 connected to each other at a lower end of the main body 110 coming into contact with the handle 130. Unlike this, the frames 111 may be separately formed without being connected to each other. Also, the frame 111 may have the form of a wire which is relatively thin or may be formed in the shape of a single wide plate.

The frame 111 of the main body 110 of the menstrual cup according to the present embodiment may be formed of a shape memory material such as a shape memory alloy and a shape memory polymer. Accordingly, the menstrual cup 100 may be easily inserted into the vagina by deforming the menstrual cup 100 using a method of bending the frame 111 to an easy-to-insert shape before insertion of the menstrual cup 100, and the menstrual cup 100 may be placed in the vagina as the frame 111 is restored to its original shape upon reaching a predetermined temperature or higher due to body temperature or a separate heat source.

In the present embodiment, since the frame 111 of the main body 110 of the menstrual cup is formed of a shape memory material, and, using this feature, the shape of the frame 111 is restored to its original shape after insertion of the menstrual cup 100, the main body 110 of the menstrual cup 100 itself does not require much elasticity. Therefore, as compared with an existing menstrual cup 100, the main body 110 may be formed with a small thickness. For example, while a main body of the existing menstrual cup is formed of silicone having a thickness of 2 mm or more, a portion excluding the protrusion 115 may have a thickness of 0.5 mm or less in the main body 110 of the menstrual cup 100 according to the present embodiment.

Meanwhile, in the process of handling the menstrual cup 100 to insert the menstrual cup 100 into the vagina after the menstrual cup 100 is deformed, heat may be transferred to the frame 111 due to body temperature or an external heat source. In the present embodiment, to prevent a temperature of the frame 111 from rising to a predetermined temperature at which shape restoration occurs, i.e., a shape restoration temperature, or more due to heat transferred to the frame 111 due to body temperature or an external heat source, a thickness of an outer wall may be formed to be larger than a thickness of an inner wall in the protrusion 115 into which the frame 111 is inserted.

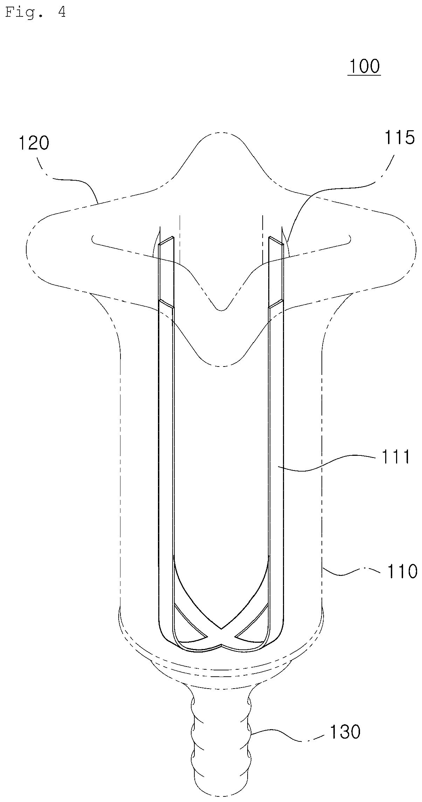

FIG. 4 is a view illustrating an example of a folded shape of the menstrual cup according to the first embodiment of the present invention which is folded to be inserted into the vagina. Referring to FIG. 4, the menstrual cup 100 according to the present embodiment may be folded into an elongated shape as if folding an umbrella. Specifically, when the frame 111 is pressed toward an inner side of the menstrual cup 100, as the frame 111 is deformed to an elongated shape, the main body 111 which is formed of thin silicone is deformed due to corrugations formed between the frames 111. Of course, the frame 111 may be deformed to a form other than that illustrated in FIG. 4.

In this way, the volume of the menstrual cup 100 is significantly reduced when the menstrual cup 100 is deformed to insert the menstrual cup 100 into the vagina, and, even when the menstrual cup 100 is deformed to an easy-to-insert shape, the menstrual cup 100 may be restored to its original shape after being inserted. Therefore, a user can easily insert the menstrual cup into the vagina with reduced feelings of repulsion or discomfort. Also, since shape restoration of the frame 111 is used instead of using elasticity of a main body of a menstrual cup as in the related art, the menstrual cup 100 may also be deformed to a shape in which an insertion aid tool such as an applicator of a tampon may be used. Thus, the menstrual cup 100 can be more easily inserted.

In the present embodiment, nitinol, which is a type of shape memory alloy, may be used as a material of the frame 111. Nitinol, which is a nickel-titanium (Ni-Ti) alloy, is the most typical shape memory alloy and is known to exhibit a shape memory effect over a wide range of temperature ranging from -240 °C to 100 °C. Also, it is generally known that deformation occurs easily at a temperature which is about 10 °C to 30 °C lower than a shape restoration temperature.

In the present embodiment, nitinol that may exhibit the shape memory effect at a temperature of about 35 °C or higher may be used. In this case, deformation may occur easily at a temperature lower than room temperature, e.g., a temperature of about 0 °C to 10 °C. After the deformation occurs at the above temperature, the shape may be restored due to the shape memory effect at body temperature.

The case in which the shape restoration temperature is similar to body temperature has been described above, but nitinol or another alloy having a different shape restoration temperature may also be used. For example, by using nitinol having a shape restoration temperature higher than body temperature, deformation may easily occur at a relatively high temperature, e.g., room temperature. In this case, the frame of the menstrual cup may be deformed at room temperature, the menstrual cup may be inserted into the vagina, and then additional heat may be supplied to the menstrual cup to restore the menstrual cup to its original shape. To this end, a separate power supply device may be disposed in the menstrual cup and configured to supply power to the frame. For example, in FIG. 3, an inner space may be provided between the handle 130 and a portion where a plurality of frames 111 are connected, and a power supply device may be disposed in the inner space and configured to supply electrical energy to the frames 111. Also, to allow a shape restoration temperature to be more quickly reached when electrical energy is supplied to the frames 111, a material with high resistance, e.g., a nichrome wire, may be attached to the frames 111.

In the present embodiment, the case in which the frame of the menstrual cup is formed of nitinol is described, but the present invention is not limited thereto, and other known shape memory materials may also be used. Since the present invention is characterized in that the menstrual cup is restored to its original shape at body temperature or a temperature close to body temperature while being inserted into the vagina, it should be self-evident to those of ordinary skill in the art that any known shape memory material may be used as long as shape restoration is possible in the above temperature conditions.

A representative embodiment of the present invention has been described above with reference to FIGS. 2 to 4. Hereinafter, various embodiments obtained by modifying the representative embodiment will be described.

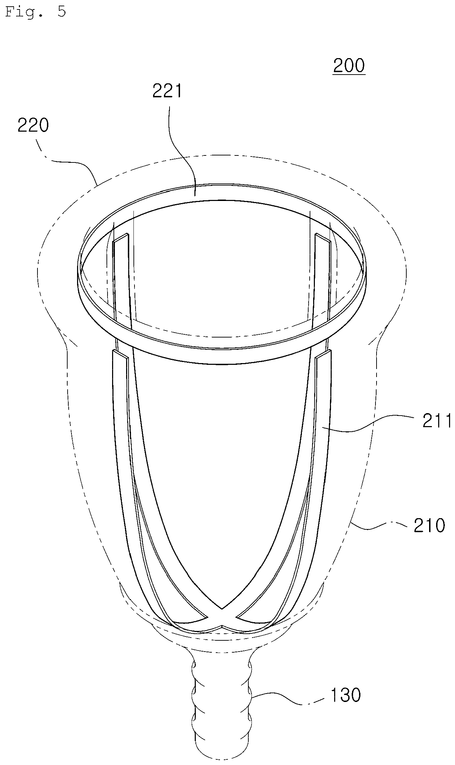

FIG. 5 is a partially see-through view of a menstrual cup according to a second embodiment of the present invention. Referring to FIG. 5, a menstrual cup 200 according to the present embodiment may include a main body 210, a rim 220, and a handle 230. The main body 210, the rim 220, and the handle 230 according to the second embodiment of the present invention may have the same outer shapes as those in the first embodiment of the present invention. Also, as in the first embodiment, the menstrual cup 200 may have the form in which a protrusion is formed on an inner wall of the main body 210 of the menstrual cup 200, and a frame 211 is inserted into the protrusion.

In the menstrual cup 200 according to the present embodiment, a ring part 221 may be formed in the rim 220 formed of silicone. The ring part 221 may be formed of a shape memory material like the frame 211 and may be formed of the same material as the frame 211. Accordingly, the ring part 221 may be deformed to an easy-to-insert shape together with the frame 211 before the menstrual cup is inserted into the vagina and then may be restored to its original shape upon reaching a shape restoration temperature or higher due to body temperature or an additional heat source after the menstrual cup is inserted.

Generally, a rim of a menstrual cup should press against the wall of the vagina so that menstrual blood does not leak out of the body. Thus, in the conventional menstrual cup, the rim is formed to be thick to have high elasticity. However, when the rim is formed to be thick and has high elasticity, it is difficult to maintain a folded shape of the menstrual cup when inserting the menstrual cup into the vagina, and, even when the menstrual cup is folded, the volume of the menstrual cup increases due to a large thickness thereof, which inevitably causes discomfort to the user.

However, according to the present embodiment, the ring part 221 formed of a shape memory material is formed in the rim 220 in addition to the frame 211 of the menstrual cup 200, and the menstrual cup 200 may be easily inserted by folding the frame 211 and the ring part 221 to an easy-to-insert shape. Also, since, rather than using elasticity of the main body 210 and the rim 220 of the menstrual cup 200, a shape restoration force of the frame 211 and the ring part 221 which are formed in the main body 210 and the rim 220, respectively, is used, the main body 210 and the rim 220 may be formed in a significantly smaller thickness as compared with the existing menstrual cup, and thus discomfort of the user may be significantly reduced.

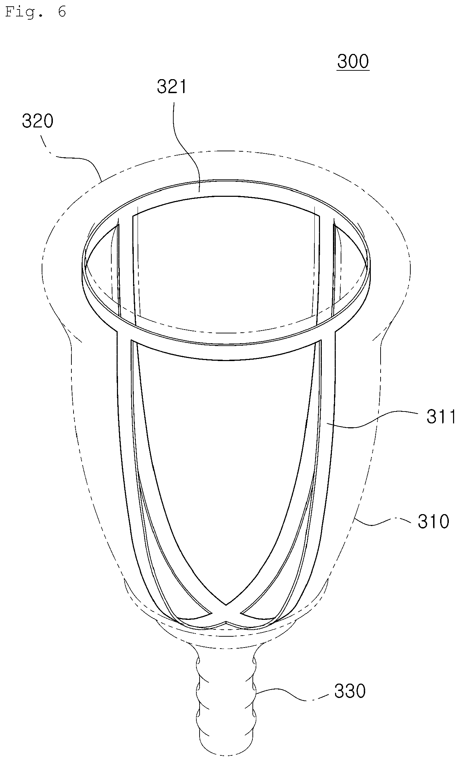

FIG. 6 is a partially see-through view of a menstrual cup according to a third embodiment of the present invention. Referring to FIG. 6, a menstrual cup 300 according to the present embodiment may include a main body 310, a rim 320, and a handle 330. The main body 310, the rim 320, and the handle 330 according to the third embodiment of the present invention may have the same outer shapes as those in the first embodiment of the present invention. Also, as in the second embodiment, a protrusion may be formed on an inner wall of the main body 310 of the menstrual cup 300, a frame 311 may be formed inside the protrusion, and a ring part 321 may be formed in the rim 320.

The frame 311 and the ring part 321 of the menstrual cup 300 according to the present embodiment may be formed of a shape memory material, and the frame 311 and the ring part 321 may be formed of the same material. Accordingly, as in the second embodiment, the frame 311 and the ring part 321 of the menstrual cup 300 may be deformed to an easy-to-insert shape before the menstrual cup 300 is inserted into the vagina and then may be restored to their original shapes upon reaching a shape restoration temperature or higher due to body temperature or an additional heat source after the menstrual cup 300 is inserted.

In the present embodiment, as illustrated in FIG. 6, the frame 311 and the ring part 321 may be integrally formed. According to the present embodiment, since a menstrual cup may be manufactured by integrally forming the frame 311 and the ring part 321 using a shape memory material and then coating the integral structure with silicone, it is possible to more easily manufacture a menstrual cup in a desired structure.

Also, since a shape restoration force of the frame 311 and the ring part 321 is used as in the previous embodiment, not only is it possible to fold the menstrual cup to an easy-to-insert shape, but it also is possible to fold the menstrual cup to a smaller volume as compared with an existing menstrual cup, thereby reducing discomfort of the user.

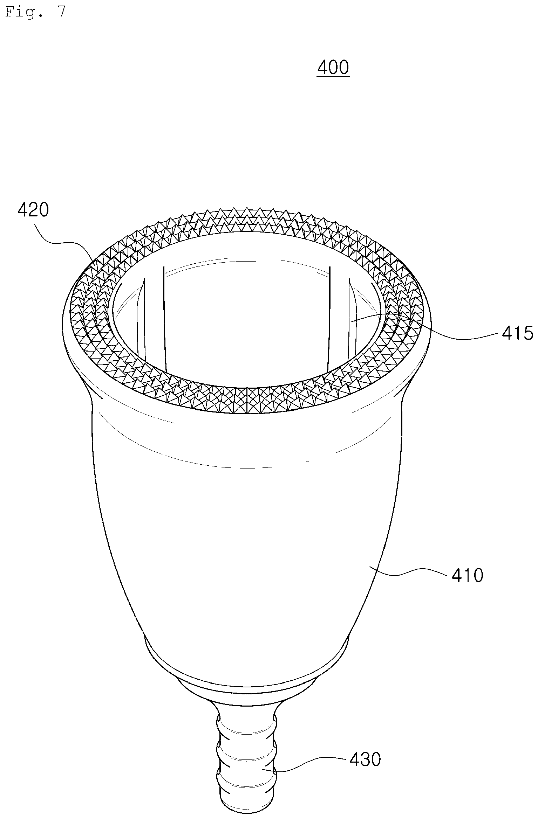

FIG. 7 is a perspective view of a menstrual cup according to a fourth embodiment of the present invention. Referring to FIG. 7, a menstrual cup 400 according to the present embodiment may include a main body 410, a rim 420, and a handle 430. The main body 410, the rim 420, and the handle 430 of the menstrual cup 400 according to the fourth embodiment of the present invention may also have the same outer shapes as those in the first embodiment of the present invention. Also, a protrusion may be formed on an inner wall of the main body 410 of the menstrual cup 400, and a frame (not illustrated) may be formed inside the protrusion.

The rim 420 of the menstrual cup 400 according to the present embodiment may have an auxetic structure. Specifically, the rim 420 of the menstrual cup 400 according to the present embodiment is formed of silicone as in the previous embodiments but has an auxetic structure having a negative Poisson's ratio with respect to an external pressure such that the rim 420 may be effectively compressed with respect to the external pressure. For example, as illustrated in FIG. 7, a plurality of three-dimensional structures having a V-shape when viewed from the top may be formed in the rim 420. Accordingly, when the rim 420 is compressed in one direction (e.g., a radial direction), the rim 420 may also be compressed in a direction perpendicular thereto (e.g., a circumferential direction) such that the rim 420 is effectively compressed.

As such, in the menstrual cup 400 according to the present embodiment, since the rim 420 may be effectively compressed and deformed, the rim 420 may, together with the frame formed of a shape memory material, contribute to deforming the menstrual cup 400 to an easy-to-insert shape and reducing the volume of the menstrual cup 400, thereby significantly reducing discomfort and a feeling of repulsion that the user experiences when inserting the menstrual cup.

The present invention has been described above using specific details, such as specific elements, and some embodiments. The embodiments are merely provided to assist in better understanding of the present invention, and the present invention is not limited to the embodiments. Those of ordinary skill in the art to which the present invention pertains may make various modifications and changes to the present invention. Therefore, the spirit of the present invention should not be defined only on the basis of the above-described embodiments, and the claims below and their equivalents should be construed as belonging to the scope of the spirit of the present invention.

## Claims

1. A menstrual cup which is easy to insert, the menstrual cup comprising:
a main body having an opening formed at one side and a storage part for accommodating menstrual blood therein formed at an inner side;
a rim formed at the opening of the main body and configured to prevent leakage of the menstrual blood by closely contacting with a wall of a vagina when the menstrual cup is inserted into the vagina; and
a frame formed inside a wall of the main body,
wherein the frame is formed of a shape memory material.

2. The menstrual cup of claim 1, further comprising a ring part formed inside the rim in a circumferential direction of the rim,
wherein the ring part is formed of a shape memory material.

3. The menstrual cup of claim 2, wherein the frame formed inside the wall of the main body and the ring part formed inside the rim are integrally formed.

4. The menstrual cup of claim 1, wherein the rim is formed of an auxetic structure having a negative Poisson's ratio.

5. The menstrual cup of claim 1, wherein the frame is formed in a longitudinal direction of a wall surface of the main body extending from the opening of the main body to the opposite side of the main body.

6. The menstrual cup of claim 1, wherein the frame is formed of nitinol.

7. The menstrual cup of claim 1, wherein the frame is restored to its original shape at body temperature.

8. The menstrual cup of claim 1, wherein the frame is deformed at room temperature and then restored to its original shape at a temperature higher than body temperature.

9. The menstrual cup of claim 8, wherein an inner space is provided at the opposite side of the opening of the main body, and a power supply device is formed in the inner space to supply electrical energy to the frame.

10. The menstrual cup of claim 1, wherein a protrusion is formed on an inner wall of the main body, and the frame is disposed inside the protrusion.

11. The menstrual cup of claim 10, wherein the protrusion is formed such that a thickness of the protrusion on an outer side of the frame is larger than a thickness of the protrusion on an inner side of the frame.
